(19) Europäisches Patentamt
European Patent Office
Office européen des brevets

(11) **EP 3 760 105 A1**

(12) **EUROPEAN PATENT APPLICATION**

(43) Date of publication:
**06.01.2021 Bulletin 2021/01**

(21) Application number: **19184582.5**

(22) Date of filing: **05.07.2019**

(51) Int Cl.:
**A61B 5/00** *(2006.01)*　　**A61M 16/01** *(2006.01)*
**A61B 5/11** *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **HELLE, Michael Günter
5656 AE Eindhoven (NL)**

• **WEISS, Steffen
5656 AE Eindhoven (NL)**
• **JOHNSON, Mark Thomas
5656 AE Eindhoven (NL)**
• **VOGTMEIER, Gereon
5656 AE Eindhoven (NL)**
• **VUPPALA, Sunil Kumar
5656 AE Eindhoven (NL)**
• **SISODIA, Rajendra Singh
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **CHEMORECEPTOR STIMULUS FOR SEDATION STATUS ASCERTAINMENT**

(57)　　A system (SYS) and related method for imaging support. The system (SYS) comprises a stimulus delivery component (SDC) configured to cause a chemoreceptor stimulus in a patient residing in or at an imaging apparatus (IA). A response measuring component (RMC) measure a response of the patient to the stimulus, and a decision logic (DL) establishes, based on the measured response, a sedation status of the patient for the purpose of imaging the patient. An imaging operation can be modified, for instance, halted if the patient is no longer sufficiently sedated.

FIG. 2

**Description**

FIELD OF THE INVENTION

The invention relates to a system for imaging support, method of training a machine learning component, to a method of imaging support, to a method of chemoreceptor stimulation for sedation status determination, to an imaging arrangement, to a computer program element, and to a computer readable medium.

BACKGROUND OF THE INVENTION

Sedative drugs are administered before or during a medical image examination, for example when the patient feels anxious or when it is necessary to ensure that the acquired images have diagnostic quality, e.g. in pediatric examination.

Moreover, some imaging approaches such as special MRI sequences rely on long image acquisition times that bear the risk of patient movement resulting in decreased image quality.

Especially in MRI, the noise level during imaging is dependent on the type of the MR sequence and is usually perceived as uncomfortable by the patient. Despite using ear protection and receiving sedative drugs, some patients show reactions during scanning, especially during the application of very noise scans.

The probability of patient movement during image acquisition can be decreased by increasing the dosage of sedative drugs or by giving an extra bolus before or during the scan. However, this increases the risk of health complications for the patient and is economically disadvantageous.

Another problem related to MRI or CT is the fact that assessment of the patient sedation status is difficult for medical staff as the patient is located during imaging inside a bore of the imager.

SUMMARY OF THE INVENTION

There may therefore be a need for a system or method to improve imaging results and to address some of the above noted shortcomings.

The object of the present invention is solved by the subject matter of the independent claims where further embodiments are incorporated in the dependent claims. It should be noted that the following described aspect of the invention equally applies to the method of training a machine learning component, to the method of imaging support, to the method of chemoreceptor stimulation for sedation status determination, to the imaging arrangement, to the computer program element, and to the computer readable medium. According to a first aspect of the invention there is provided a system for imaging support, comprising:

- a stimulus delivery component configured to cause a chemoreceptor stimulus in a patient residing in or at an imaging apparatus;
- a response measuring component configured to measure a response of the patient to the stimulus; and
- a decision logic configured to establish, based on the measured response, a sedation status of the patient for the purpose of imaging the patient.

In embodiments, the decision logic is further to provide, based on the established sedation status, any one or more of: i) a control signal to control the imaging apparatus and/or ii) an indication on the sedation status and/or on how to operate the imaging apparatus. The controlling may include any one of more of: halting an ongoing imaging operation, initiating an imaging operation, delaying a planned imaging operation. *"Imaging operation"* as used herein is the period where detector of the imaging apparatus acquires signals that are convertible into imagery. The imager may be automatically so controlled, without user input from the imager's console, or the control options are displayed on the display device to inform the user on how to operate the imager, that is, to halt or commence the imaging operation, or to delay, preferably displaying the delay time, for example as a countdown time or other.

In embodiments, the causing by the stimulus delivery component of the stimulus comprises any one or more of: i) providing a substance to the patient capable of stimulating a chemoreceptor of the patient or ii) applying an electrical signal (voltage or current) to a chemoreceptor of the patient. The substance or chemical may be in liquid form or may be gas form or in form of an aerosol. The substance may be a tastant or odorant.

In embodiments, the decision logic comprises a pre-trained machine learning component (MLC). The MLC maybe based on a (deep) neural network (NN) or other, such as support vector machines (SVM), etc.

In embodiments, the stimulus delivery component comprises any one or more of: i) a portable device configured for oral placement and configured to electrically induce a taste sensation in a patient by having electrodes of the device in contact with at least a part of the patient's tongue when so placed, and configured to apply a voltage across the electrodes driven by a power source ii) a portable device configured for oral placement, having at least one container including a tastant or odorant, the device configured to release a quantum of said tastant upon the device receiving a trigger signal, iii) a tube arrangement in fluid communication with a container including a tastant or odorant, and a pump configured to pump a quantum of the tastant or odorant from the container, through the tube arrangement, to a terminal end thereof, so as to dispense the quantum of tastant or odorant at the patients mouth or nose, iv) a jet-delivery system including a nozzle through which an odorant is delivered as an aerosol. The tube arrangement in iii) may include fixation means (straps, fasteners, ect) for attachment and alignment of the terminal end of the tube to ensure efficient delivery.

The nozzle may be integrated into a face mask, or into the imaging apparatus (eg, its bore), into a coil arrangement of an MRI apparatus, into ear defenders, into a patient microphone, and others.

In embodiments, the power source is based on inductive coupling. In embodiments, the power source includes an induction loop. The powering may be done by using an external radiofrequency signal source, such as that of the imaging apparatus itself if this is an MRI imager. This allows an efficient design with few items of extra equipment.

In embodiments, the response measuring component comprises a sensor arrangement capable of measuring any one or more in relation to the patient: i) a galvanic skin response ("GSR") signal, ii) a facial gesture, iii) a body gesture, iv) an uttered sound, v) a vital sign.

Accordingly, the sensor may comprise an optical or non-optical camera for still imagery or video coupled to an image recognition facility capable of detecting facial expression, body movement in general or other. Instead of using a dedicated camera, the imaging apparatus itself may be used, for instance in a low does scout scan or MRI measurement with motion detection or other of the acquired imagery. Alternatively, a neuro scan is done to assess the response in terms of brain activity.

In addition or instead, a GSR sensor may be used or other probes to measure blood pressure, pulse, oxygen saturation (eg, spirometer) etc. The GSR sensor or vital sensor may be integrated into face mask wearable by the patient during the imaging session.

In yet other embodiment, a microphone is used to capture utterances (in particular exclamations) and use voice recognition to establish sedation status or level.

In embodiments, the stimulus is caused in a sequence of different intensities. The different stimulation intensities may be effected by using, over time, different tastants or odorants, and/or volumes thereof and/or concentrations of the same or of different tastants or odorants. Alternatively, the sequence of different intensities is caused by varying the applied electrical signal. Causing the stimulus in varying intensities allows establishing a sedation level rather than merely establishing a binary status of sufficient sedation or insufficient sedation. However, this binary option of establishing sufficient versus insufficient sedation status is also envisaged herein in embodiments.

According to a further aspect of the invention there is provided a method of training the machine learning component ("MLC"). Using machine learning allows for a reliable determination of sedation status/level.

In one embodiment, a supervised ML scheme is used for the calibration method, in which case the method may comprise, in embodiments, the steps of:

- receiving training data comprising i) patient data and a target comprising a combination of tastant or odorant and/or ii) a response measurement and as a target a classification into sufficient or insufficient sedation or into a plurality of sedation levels.
- applying the training data to a machine learning model to obtain a training output data;
- comparing the training output with the target; and
- adjusting parameters of the model based on a deviation between the target and the training output.

However, unsupervised learning may also be used in which case there is no or not necessarily a target, that is the training data is not generally structured as described above.

According to a further aspect of the invention there is provided a method of imaging support, comprising the steps of:

- causing a chemoreceptor stimulus in a patient residing in or at an imaging apparatus;
- measuring a response of the patient to the stimulus; and
- establishing, based on the measured response, a sedation status of the patient for the purpose of imaging the patient.

According to a further aspect there is provided a method for chemoreceptor stimulus based sedation control, comprising the steps of:

- receiving training data comprising patient data and stimulant data, the stimulant data comprising a combination of different base tastes; and
- applying a machine learning algorithm to the training data to learn a relationship between the patient data and the stimulant data.

These steps may be understood to define a calibration phase.

The machine learning algorithm may include supervised and unsupervised learning, neural network NN-based, SVM, clustering algorithms such $k$-means, etc.

In embodiments, the method further comprises:

- predicting, based on the learned relationship, new stimulant data given new patient data of a patient to be imaged; and
- stimulating the patient based on the new stimulant data to establish a sedation status of the patient.

According to a further aspect there is provided an imaging arrangement, comprising:

- a system of any one of above mentioned embodiments;
- the imaging apparatus.

According to a further aspect there is provided a computer program element, which, when being executed by at least one processing unit, is adapted to cause the processing unit to perform any of the above mentioned methods.

According to a further aspect there is provided a computer readable medium having stored thereon the program element.

With the proposed systems and methods, better imaging results can be achieved by reliably establishing a sedation status/level of a patient and to commence or continue image acquisition only once the patient is sufficiently sedated. The sedation status is established based on chemoreceptor (taste or smell receptor cells) stimulation. Furthermore, the proposed system may be used to administer just the right amount of sedative for the imaging task, not more and net less. This promotes patient health and keeps costs down.

Preferably, the stimulus delivery component is operated to deliver the stimulant before the imaging acquisition, eg MR sequencing, is to start. In other words, stimulation occurs before a signal source of the imager is energized to so acquire the desired imagery. More specifically, an imaging session may then be divided into three phases: in the first phase the patient is prepared and positioned in or at the imaging apparatus, in a follow-up phase the sedative is administered. In the third phase, after administration or during administration of the sedative, the stimulant is delivered by the stimulant delivery component. During or after stimulant delivery, the response is measured by the response measuring component. The decision logic then measures the response signal to determine the level of sedation. If the sedation level is high enough, the actual image acquisition may then commence. Monitoring of the response through the response monitoring component is preferably ongoing during the image acquisition to so detect instants where the patient is reawakening. If required, it may be indicated to the user to halt the imaging acquisition if the sedation level drops. Alternatively, decision logic may autonomously stop the imaging procedure and optionally indicate this by an alert signal to the user.

Definitions

*"user"* a referred to herein is medical personnel at least partly involved in an administrative or organizational manner in the imaging procedure.

*"patient"* is a person, or in veterinary settings, an animal (in particular a mammal), who is be imaged.

*"sedation status/level"* is a state of the patient, established by analysis of the response, and is an indication to what extent the patient is sedated. The patient is considered sufficiently sedated for imaging purposes, if there is no patient motion (other than motion induced by heartbeat and/or breathing), and/or whether the patient motion is under a threshold. The sedation status may be binary, or may be graduated into more than two levels. *"sedation status"* and *"sedation level"* is used interchangeably herein.

BRIEF DESCRIPTION OF THE DRAWINGS

Exemplary embodiments of the invention will now be described with reference to the following drawings, which are not to scale, wherein:

Fig. 1 shows a block diagram of a system to support an imaging operation;
Fig. 2 shows a stimulus delivery component according to one embodiment;
Fig. 3 shows further embodiments of stimulus delivery components;
Fig. 4 is a flow chart of a method of imaging support;
Fig. 5 is a flow chart of a method of training a machine learning model;
Fig. 6 is exemplary training imagery that represents different facial expressions in response to stimuli;
Fig. 7 is a flow chart for a method for chemoreceptor stimulus based sedation level determination; and
Fig. 8 shows a machine learning model envisaged in embodiments.

DETAILED DESCRIPTION OF EMBODIMENTS

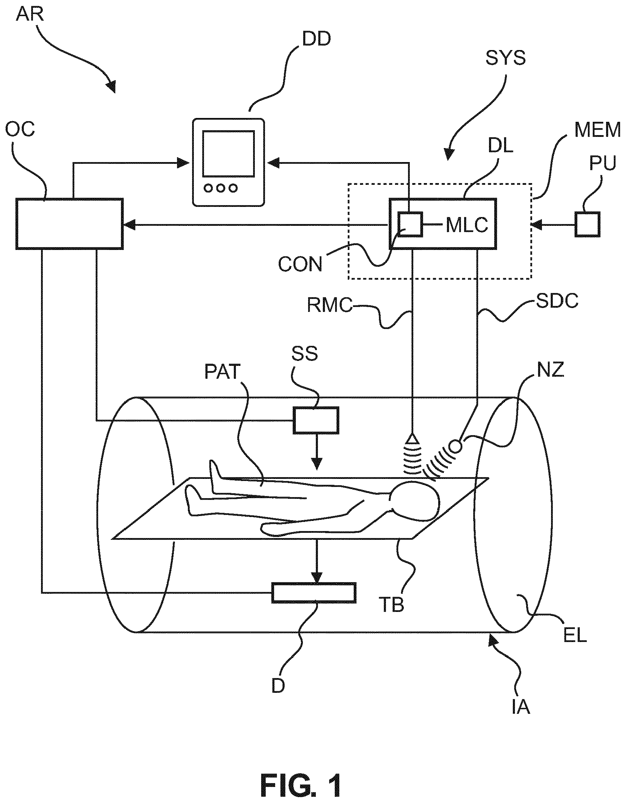

With reference to Fig. 1, there is shown a schematic block diagram of an imaging arrangement AR. The imaging arrangement AR includes an imaging apparatus IA configured to acquire medical imagery of a patient PAT. The patient may reside on an examination table TB. The imagery may be displayed in a display device DD to support therapy or diagnosis, or may be forwarded for storage for later "reading", or may be otherwise processed. The imaging arrangement AR further comprises a computerized system SYS configured to support an imaging procedure.

In some imaging procedures it is important that the patient does not move whilst the imager IA operates to acquire the imagery. Patient motion during image acquisition can severely degrade image quality and can even render imagery completely useless requiring imaging re-takes. Image retakes may however pose a health hazard in particular with imaging apparatuses where ionizing radiation is used such as in CT or other x-ray based imaging modalities all envisaged herein. Unnecessary re-takes are to be avoided to keep x-ray dose exposure down and ensure smooth workflow without delays.

To promote stationery of the patient PAT during imaging, sometimes a sedative is administered to the patient, either orally or intravenously. However, different patients react differently to the sedative and it may not always be clear whether the patient is sufficiently sedated to ensure the desired image quality.

The proposed computerized system SYS helps support the imaging procedure in establishing whether the patient has been sufficiently sedated or not. Operation of the computerized support system SYS harnesses the patient's chemoreceptor system. Responses of the patient's chemoreceptor system to targeted stimuli have been found to be reliable tokens to establish sedation level of the patient.

The chemoreceptor system of the patient, and indeed in all mammals, includes a sense of smell and a sense of taste, the olfactory system and the gustatory system respectively. Outlining first some biological facts about the chemoreceptor system before explaining operation of the computerized system SYS for imaging support further, the olfactory system comprises specialized receptor cells located in a patch of mucus membrane that lines the roof of the nose. Chemicals, such as liquid suspended or airborne molecules or particles, generally called odorants, may enter patient's nose and interact with the said sensory cells. The interaction elicits electrical signals that are conducted through a nerve cell network into the brain where the sensation of smell is established. The gustatory system is formed by specialized receptor cells called taste buds. Some taste buds are located on the patient's tongue, or the soft palate, and in or around other regions of the mouth cavity. A class of chemicals, generally referred to as tastants, in generally suspended in liquid but also airborne, enter the mouth cavity and contact the tongue and then interact with the taste buds. Such interaction will elicit electrical signals that are conducted again through a corresponding nervous cell network into the brain to establish the sensation of taste. In other words, as with other parts of the nervous system, information exchange in the chemoreceptor system is based on electrical signaling.

Referring now back to the computerized system SYS as proposed herein, this includes a stimulus delivery component SDC and a response measuring component RMC. Broadly, the stimulus delivery component SDC causes, in a manner to be explained in more detail, below a chemoreceptive stimulus by interaction with the patient's chemoreceptor system. The chemoreceptor may either be a taste bud and/or a sensory cell of the olfactory system. A response by the chemoreceptor system to the stimuli is then picked up by the response measuring component RMC. The response is then analyzed by a decision logic DL.

Broadly, the decision logic is to establish based on the measured response the sedation status or level of the patient and this information may then be used for imaging purposes in respect of the patient. The system SYS is envisaged to operate during an imaging session, that is, whilst the patient is in or at the imaging apparatus in the imaging room. The response measuring component and the stimuli delivery component may be integrated in one of the same unity or may be arranged separately.

The decision logic DL may run in hardware or software or in a combination thereof on a computer unit PU. The decision logic may be resident in one or more memories MEM. The system SYS may be integrated into an operator console OC of the imaging apparatus IA or may be arranged in a different processing unit PU different from the operator console, such as a work station, or a mobile computing unit (smartphone, tablet, mobile computer (laptop, notebook, subnotebook etc) or other. The decision logic DL preferably checks whether the patient has been sufficiently sedated. Such may be the case if the response picked up by the response measuring component is below a threshold relatively. As will be explained in more detail below, the stimuli are carefully chosen in embodiments to ensure that a response of a certain magnitude can be expected if the patient is indeed not sufficiently sedated. If this response is not achieved at the expected magnitude, a conclusion is made that the patient is indeed sufficiently sedated.

In embodiments, the conclusion reached by the decision logic in relation to the sedation level is displayed on the display device DD. For instance, a confirmatory "all clear" message maybe displayed on the display device DD to indicate to the user of the imaging apparatus that the patient has been indeed sufficiently sedated. In other embodiments however nothing is indicated on the monitor unless the decision logic comes to the conclusion that the patient has not been sufficiently sedated and in which case an alert message is displayed on the display device DD, or a sensory indication is provided such as sounding out an alarm, or activating an alarm lamp, etc.

In addition to, or instead of, merely providing an above described indication, the decision logic may be operable to affect the imaging procedure. Specifically, the decision logic may be equipped with interface circuitry that allows interacting with the imager's IA operator console to actively influence the imaging procedure, in particular in fully automatic, autonomous imagers IA. More specifically, the decision logic may send a control signal through a control interface CON to the operator console to interrupt or halt an ongoing procedure if the decision logic comes to the conclusion that the patient is not or is no longer sufficiently sedated. In addition or instead, if the decision logic establishes that the patient is not sufficiently sedated it may override a request by the operator or user to proceed or initiate an imaging procedure. In other words, the decision logic may send a signal to the operator console to block a request of the user to start or initiate the imaging procedure because the patient is not sufficiently sedated. The decision logic may block the imaging procedure to go forward until the decision logic establishes, based on further responses picked up by the response measuring component, that the patient is or is again sufficiently sedated. Thus the decision logic may delay a planned imaging procedure until the patient is found to be sufficiently sedated.

Turning now first to the imaging apparatus IA before explaining the computerized system, a range of different modalities are envisaged herein for use with the proposed imaging support system SYS. Specifically, the imaging apparatus IA envisaged herein in embodiments includes emission based imaging or transmission based imaging but also includes ultrasound imaging or magnetic resonance imaging MRI.

In embodiments, the imaging apparatus IA is configured to acquire medical imagery of internal structures of the patient PAT. The medical imagery may reveal inner organs of the patient. Alternatively, instead of anatomic-structural imaging such as in X-ray or MRI, functional imaging is also envisaged. Functional imaging allows visualizing, for instance, metabolic activity as in some types of nuclear emission imaging, such as SPECT or PET. In either case, the medical imagery may assist in therapy or diagnosis of the patient PAT.

In broad terms, and turning first to the imaging apparatus IA in more detail, this includes, in embodiments, equipment to generate an interrogating imaging signal. The interrogating imaging signal interacts with tissue inside the patient. Patient tissue modifies the interrogating signal and causes a modified signal. The so modified signal encodes information about the internal structures, anatomies, functional activity, etc, of the patient. The modified signal can be detected by a detector unit D. The signals detected at the detector unit D may be converted by circuitry into imagery that may be displayed on the display device DD.

One embodiment for transmission imaging in particular envisaged herein is X-ray based imaging such as radiography, C- or U-arm imaging or computed tomography CT imaging ("CT scanner"). In these embodiments, the interrogating imaging signal is an x-ray beam XB. In this embodiment, the equipment to generate the X-ray beam includes an X-ray source XR, such as an X-ray tube. The X-ray source is energizable to release the x-ray beam in an otherwise known manner. The x-ray beam passes through the patient, is then modified, in particular attenuated, and the so modified beam is then detected at an x-ray sensitive detector D.

The x-ray sensitive detector D is arranged opposite the x-ray source to form an imaging region in between the two where the patient resides during the imaging. The term "imaging" as used herein is the period throughput which the interrogating imaging signal, such as the x-ray beam, passes through the patient and is detected at the detector D. Intensity data registered at the detector D can then be processed by suitable circuitry into internal anatomical or functional images displayable on the associated display device DD. For instance, in CT cross sectional x-ray images can be obtained from the detected X-ray intensities. The imagery may be rendered for visualization on the display device DD to assist clinical personnel. In the emission embodiment, the equipment to generate the interrogating imaging signal resides within the patient, after suitable administration prior to imaging. The equipment may include a suitable radioactive tracer substance. The tracer substance interacts with patient tissue from within, then egresses the patient and is then detected by detectors arranged outside the patient, at last partially surrounding the patient, as is in PET or SPECT and similar imaging techniques.

In MRI, the detecting unit D comprises one or more radio frequency coils that are capable of picking up radio wave signals. The radio wave signals are emitted by disturbed protons in patient tissue when relaxing back into alignment with a strong magnetic field generated by the MRI imager during the imaging.

Loud banging noise can be heard during NMR imaging caused by vibrations in the NMR imager's gradient coils. Especially at the onset if imaging, when the sound suddenly sets in, this may cause the patient to startle, resulting in involuntary jerky motion. This may lead to motion artifacts in the imagery. This high, and sudden, sound level could cause great discomfort to the patient making the administration of the sedative advisable, in particular in MRI imaging.

Some of the described imaging apparatus IA embodiments, but not necessarily all, may include a physical enclosure EL that surrounds the patient during imaging. The patient resides within the enclosure EL during imaging, possibly lying or sitting on a support such as an examination table TB inside the enclosure. CT scanners and MRI scanners may include such enclosures EL arranged as a bore. The bore is relatively tight and does not leave much space for the patient to move. Being confined for potentially prolonged time in the right enclosure may also cause patient discomfort, which, again may call sedative administration in particular for claustrophobically inclined patients.

With continued reference to the computerized system SYS for imaging support, the stimulus delivery component SDC may now be explained in more detail with reference to a number of different embodiments each of which envisaged herein in isolation, or in combination or any sub-combination.

In one embodiment, and as is indicated in Fig. 1, the stimulus delivery component SDC may include jet spray system with a nozzle NZ integrated at least in part into imaging apparatus, in particular in the imager IA's bore EL, eg in a wall thereof. In addition or instead, the nozzle NZ may be integrated in an MR receive coil, such as head coil, or an anterior coil array. Other embodiments include integration of the nozzle NZ into an ear protection device such as an ear fender that some patients may choose to wear in MR imaging to muffle the above mentioned noise caused by the vibrating MRI coils. Another embodiment envisages integration into a patient communication device such as microphone that allows the patient to remotely communicate with staff whilst the patient is in the imaging room, in particular whilst the patient is in the imager's IA bore EL.

An odorant or tastant is drawn from a reservoir and can be injected into the bore EL through the nozzle as an aerosol or gas, with a spray jet projected towards the patient's face, in particular towards the patient's nose. The nozzle NZ is preferably articulated and movably arranged so that it can be aligned with a given position of the patient's nose. The nozzle NZ maybe mounted outside the bore EL, anywhere in the examination room. For instance, the nozzle may be ceiling or wall mounted, or may be mounted on a gantry or is otherwise suitably spatially arranged to that the odorant can be projected preferably towards the patient's face, in particular nose. Air or other gas may be used as propellant. Delivery of tastant or odorant through the proposed jet-spray system is efficient in there is no requirement for the patient to open their mouth widely.

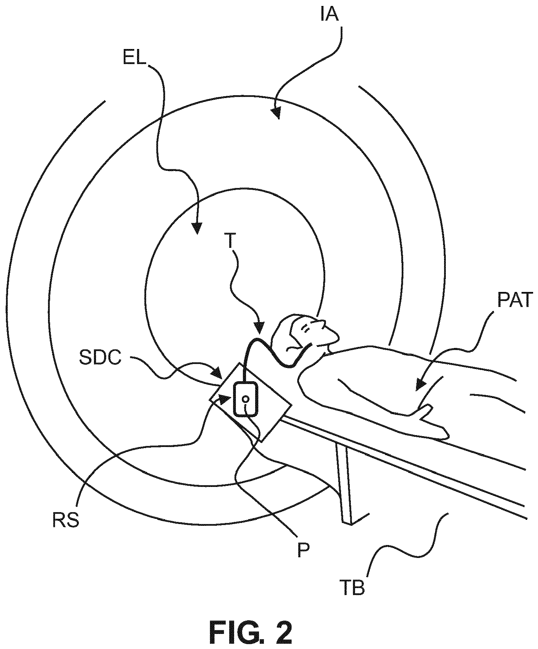

With reference to Fig. 2, this shows another embodiment of the stimulus delivery component SDC for odorant or taste delivery. More specifically in embodiments the stimulus delivery component SDC comprises a container RS that includes a chemical, the tastant or odorant. The one or more containers are in fluid communication with a tube that is guided to the patient's nose or mouth. The stimulus delivery component SDC may further comprises a pump P that is operable by user request to expel odorant or tastant from the reservoir through the tube towards the patient's mouth or nose. A fixation means may be provided such as a strap or otherwise, that allows the terminal end of the tube to be positioned close to the patient's nose.

For tastant delivery the terminal portion of the tube may extend up to the patient's mouth, or may extend at least partially thereinto. The tube T may terminate in a crooked or hooked portion, so as to be engageable with the corner of the patient's mouth. Suitable valve means may be provided to ensure the odorant or tastant is safely contained in the container and only expelled upon request. The pump P and an actuator to open or close the valve may be communicatively coupled through a wired, or preferably wireless, connection with the operator console OC or with any other remote control device. In embodiments of Fig. 2, the tube may terminate into a nozzle and delivery may be through gas (eg, air) propelled jet-spray as described above.

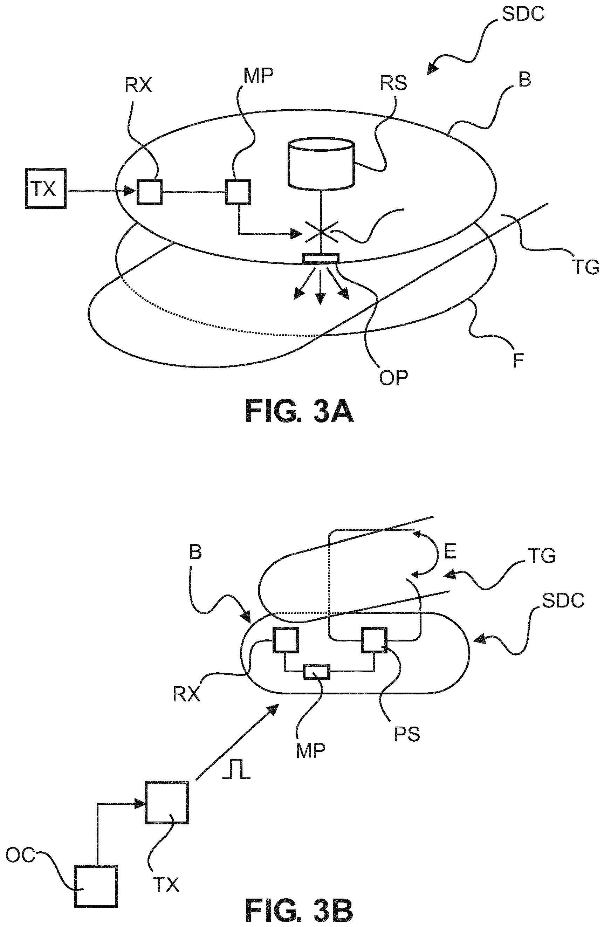

Reference is now made to Figs.3A, B which show two embodiments for portable stimulus delivery components SDC. In these embodiments, the stimulus delivery component SDC is arranged as mobile, portable device that is dimensioned to be placed into the patient's mouth.

Referring first to the embodiment shown in Fig. 3A, this includes a reservoir RS. Each reservoir holds a volume of tastant, preferably in liquid form. The one or more reservoirs RS are embedded into a body B formed from a bio-compatible material such as glass, plastic or other. The overall dimension of the portable stimulus delivery component is small enough to be placed into patient's mouth, but not too small to avoid choking. The portable stimulus delivery component may include a fixation means F that allows fixing the device SDC onto the patient's tongue TG.

Tastant in the reservoir RS can be expelled through an opening OP on actuation of an opening valve VL. The valve VL is controlled by a micro-processor MP which in turns responds to a trigger signal received by a receiver component RX. The receiver RX receives the trigger signal from a transmitter component TX that may be located in the mentioned remote control device or may be located in the operator console OC, or elsewhere. The transmitter may be user controlled or the transmitter sends the control signal to open or close the valve VL on instruction from the decision logic DL.

In the embodiment shown in Fig. 3B, when the device SDC is mounted on tongue TG, the opening OP is located opposite and above the surface of the patient's tongue. In other words, the tastant can be expelled from the reservoir simply through gravity. Other solutions may include a pump unit P as discussed previously in Fig. 2 in which case the unit SDC may be positioned under the tongue, with a tube section running from the opening OP around the tongue to dispense the odorant on top of the tongue. However, the arrangement in Figure 3A as a tongue" back-pack" is preferred as no active pumping unit P is required thus making the device simpler and less prone to malfunction and cheaper to produce. In yet other embodiments, the opening is not necessarily directed towards the tongue as a sufficient taste sensation may still be produced if the tastant is release somewhere into the patient's mouth.

The above discussed embodiment of the stimulus delivery component SDC in 3A were configured to release a "true" tastant and odorant, that is, a chemical. These embodiments maybe referred to herein a "natural" stimulus delivery components. However, "artificial" stimulus delivery components are also envisaged here and will now be discussed with reference to Fig. 3B. In these embodiments there is no chemical dispensed, or not necessarily so. Rather, in these embodiments, the electrical nature of the signal nervous pathway is harnessed. In other words, the artificial chemoreceptor stimulus device SDC is configured to apply an electrical signal directly to the sensory cells, in particular taste buds, to so elicit a sensory taste response.

In more detail (with like reference signals indicating like components in Fig. 3A discussed previously), the component includes a power source PS coupled to a pair of electrodes E. As shown in Fig. 3B, the electrodes form an open loop through which the tongue may pass to so affix the stimulus delivery component to the tongue. However, a separate dedicated fixation component (not shown in Fig. 3B), similar to the one in Fig. 3A may also be used in addition.

After receiving the trigger signal at the receiver RX from the transmitter TX, micro-processor MP operates to instruct the power source PS to apply a voltage across the electrodes. This will then elicit a response at certain taste buds to so cause in the patient a sensation of taste.

Referring now back to the embodiment in Fig. 3A, the reservoir RS may include a number of compartments, each including a different tastant or the same tastant in different concentrations. The different compartments may discharge into a common feed line which then connects into the valve VL and the opening OP. Each compartment is individually addressable through a local valve(let) (not shown), to release a desired quantum of the respective tastant from the respective compartment. A mixture of tastants can be produced. The mixture is collected in the common feed line and is then dispensed through the opening into the patient's mouth. In this manner, different types of taste sensations may be elicited at different levels. This may be useful in embodiments where different patient characteristics may respond differently to tastants. For each patient, the correct tastant combination can be chosen and dispensed. The tastant combination is a correct one for a given patient, if a sufficiently high response level can be expected, given the patient is not sufficiently sedated.

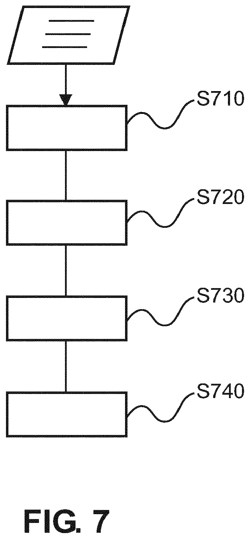

The question of what constitutes a sufficient level of response and which kind of stimulus should be administered to a given patient will be discussed in more detail below at Fig. 7.

A range of different tastes may also be cause by the "artificial taste" generator embodiment of Fig. 3B. Specifically, rather than using a single electrode pair E, a matrix of electrode pairs may be arranged to cover an area of the tongue. Each electrode pair in the matrix is individually addressable at different voltages, so that different taste bud can be excited at the same time with different voltages. This allows artificially generating practically any taste sensation.

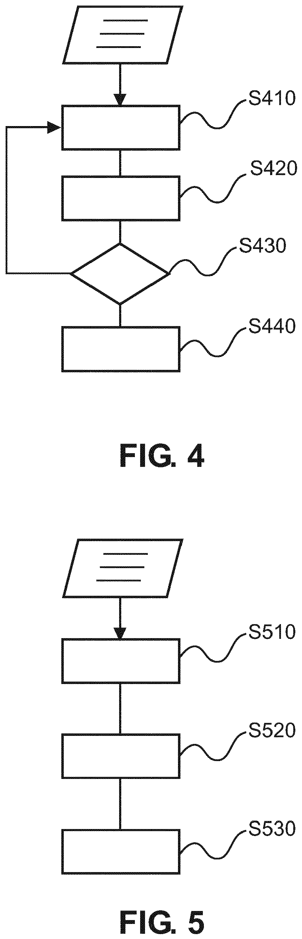

With continued reference to Fig. 3, the power source PS in Fig. B and, if applicable, in Fig. 4A to drive the pump if required, may be provided as an on-board battery module. However, the device SDC may also be configured to be power-driven externally. The power source may be located externally to the device SDC, and connected thereto through a wired, or, more preferably, through a wires connection. In particular, in wireless embodiments, radio frequency signals generated by the interrogator coils of an MRI imager IA may be used to induce a current in a secondary coil integrated into the portable device SDC. In other words, the imager itself may be used to control the power supplied to, and hence the operation of, the induction driven portable device SDC. The MRI imager may hence be operated, before or in between image acquisitions, in a mode to drive the portable device SDC to cause the simulation.

More particularly, a size of the device can be reduced by using the inductive coupling to provide the required operating voltage. In basic arrangements, the device may only consist of a flexible plastic foil with an embedded wire-loop that makes contact to an electronic circuit and finally to the two electrodes E at the surface of the foil. Due to the limited size of the device, high frequency transmission of power is proposed, followed by rectification and modulation to put out about 100Hz as required for stimulation. The device with the inductive coupler is cheap to produce and may be used as a disposable.

For application in MR imaging, the RF transmission path of the MR system can be used to generate the magnetic field required for induction with full control by the scan software. RF pulses with a frequency that largely differ from the MR imaging bandwidth can be used to avoid any interference with MR imaging.

The induced voltage can be estimated as $U = n\pi(d/2)^2 B_1\, 2\, \pi f$, with $n$ = number of loops, $d$ = diameter of loop, $B_1$ = amplitude of magnetic field of RF pulse, $f$ = carrier frequency of RF pulse.

For example, with $n = 4$, $d = 2$cm, $B_1 = 10\mu$T, $f$= 64MHz, a voltage U = 5V can be induced that results in more than 2V after rectification and frequency modulation. The received power is sufficient to drive the electronic circuit to perform rectification and frequency modulation.

The power requirements are modest so that a non-resonant receive circuit can be used in the device, thus RF safety can be maintained. Alternatively, or in non-MR applications, separate RF transmit coils positioned next to the head of the patient, for example integrated in the patient support TB) may be used instead of the MR body coil. This provides the additional degree of freedom to increase the carrier voltage enabling even smaller devices.

Other embodiments of the portable, mouth deployable stimulus delivery component include techniques used in in-vitro drug delivery. In embodiments, a micro-bubble packaged taste stimulus, preferably encapsulated in a gum or gel encapsulation B, can be placed into patient's mouth. Tastant delivery may then be effected by blasting the encapsulation with ultrasound energy, such as provided by a High frequency focused ultrasound (HIFU) system to perforate or rupture bubbles to so release the tastant. Specifically, a micro-bubble, when exposed to such ultrasound energy, starts heating up which then results in bursting of the air bubble and hence the releasing of the taste stimulus.

Referring back to the embodiment in Fig. 1 where delivery is through the nozzle NZ, this may be modified in embodiments by having, instead of the nozzle, an outlet from a reservoir to which is attached a feed line that terminates into a face mask that is placed on the patient's face. The patient is wearing the mask during the imaging session. The mask may also be used to deliver the sedative. A separate, different channel in the mask may form a delivery channel through which the tastant or odorant is delivered.

It will be understood that each of the above described embodiments for the stimulus delivery component SDC may be used singly in isolation or may be used in combination or sub-combination. In general, the delivery of the tastant or odorant, that is the stimulant, is preferably controlled by the operator but may also be controlled fully or semiautomatically by the decision logic DL or other control entity. The decision logic DL may be part of a robotic system as envisaged for autonomous imaging equipment.

In embodiments it is envisaged to deliver the stimulant (that is, the odorant or tastant) not in a single spurt at a given one time, but through a series of quanta at different times during the imaging session, with varying levels of concentration or intensity or mass/volume. This gives rise to a time curve, referred to herein as the "delivery curve". Such repeated delivery of quanta of stimulants over time may be useful during sedation level/status monitoring in an on-going imaging session. In particular, higher intensity levels of stimulant delivery alternate with lower concentration or intensity stimulant delivery during the course of the imaging session. More particularly, an over time, saw-tooth profile of the delivery curve is envisaged herein in embodiments. Other periodic or non-periodic profiles, sinusoidal or not may be used. This avoids the patient getting used to the stimulant thereby running the risk of the decision logic making a wrong decision. Such errors include concluding that the patient is sedated although he is not or, vice versa, that the patient is not sufficiently sedated although he or she is. Both error types are undesirable.

In addition or instead of the above, administering the stimulant at varying intensities over time, eg in sawtooth profile or other, allows to establish a depth of sedation, rather than merely a binary status of whether the sedation is sufficient or not. A higher threshold before the patient responds would indicate a higher level of sedation. The sedation level may be monitored periodically by operation of the response measurement component RMC, to indicate if sedation is deepening or wearing off.

Administering the tastant or odorant at different levels of intensity (by varying volume and/or intensity or type of stimulant) may be implemented by the above mentioned multi-compartment embodiment of the in situ device as discussed in Fig. 3A or B. In these embodiments, each compartment may be pre-loaded with tastants at different concentrations and these are then dispensed in a time sequence accordingly to implement the oscillating intensity/concentration curve. Alternatively, the embodiment in Fig. 2 maybe used with multiple outside containers each including a tastant or odorant at a different intensity. A switch in the feed line from the container to the dispensing device (eg, the face mask or nozzle NZ) may then be used to implement the variation in time of the intensity/concentration curve.

All of the above said is of equal application to the embodiment in Fig. 3B where the stimulus is elicited artificially by application of the electric voltage to the taste buds as explained above.

Turning now in more detail to the response measuring component RMC, a number of different embodiments are envisaged.

In some embodiments, the response measuring component RMC is image based. It includes a camera or video component. Measurement of the response or reaction of the patient to the administered stimulus is then image based. Specifically, after stimulus delivery, imagery of the patient is acquired by the imaging component of the RMC and this image or stream of images is then analyzed by an image analyzer component of the decision logic. In embodiments, the image analyzer analyzes the one or more monitoring images acquired by the RMC to determine a facial expression of the patient for instance, or a change of facial expression, or indeed may be determine more generally patient movement, not necessarily confined to the face. For instance, patient may move their head, their arm or leg as a token of response.

If the patient motion exceeds a certain predefined level, this may be indicative that the sedation level is too low. Preferably, the facial expression of the patient is established as this has been found to be a good indicator for the sedation level. Although slightly different for each individual, exposure to, for example, pungent smells or tastes for example are known to induce characteristic facial expression to as tokens for relish or aversion and disgust.

The camera component of image based embodiments of the RMC may be a dedicated camera arranged in the imaging room or at the imaging apparatus so that the relevant body part, such as the face, can be kept in the field-of-view of the camera or video component. Specifically, in embodiments, the camera or video component may be integrated into the imager's bore. However, in alternative embodiments there is no such dedicated imaging equipment, but it is the imaging apparatus itself that is used to acquire, in a scout imaging or a monitoring imaging phase, imagery of the patient for the purpose of establishing the patient's response to the administered stimulus. For example, in CT embodiment a scout scan may be done with a very low dose, lower than for the subsequent image acquisition for diagnostic or therapeutic purposes. Equally, in MRI the imager may be operated to image the patient after stimulant delivery to establish, by image analysis of the imagery, patient motion or facial expression. The image analyzer may be configured to scan the acquired imager for motion artifacts to establish the sedation level. Alternatively, still a neuro-imaging may be done, preferably functional imaging, to capture brain activity and to have the decision logic DL analyze the functional imagery for patterns that correlate with sedation status. fMRI or nuclear imaging such as PET/SPECT are in envisaged for this.

In other embodiments, the RMC is not necessarily image-based but is configured to acquire one or more vital signs such as blood pressure, heart frequency etc, and the decision logic DL establishes the sedation level based on patterns in these measurements. In yet other embodiments, the RMC includes probes to measure a galvanic skin response ("GSR") signal of the patient. The probes may be integrated into the above mentioned facial mask for instance.

In addition or instead to GSR measurements, optical sensors urged into contact with the patient's skin may be used to detect the response to the stimulant. These optical sensors may also be integrated in addition or instead to the above mentioned GSR probes into the face mask, if any.

In yet a further embodiment, the response measuring component RMC includes one or more microphones suitably arranged in the imager's bore or around the imager, to pick up speech signals from the patient. The speech signals are analyzed by a speech recognition functionality of the decision logic. The speech recognition functionality is configured to identify certain speech that may indicate that the patient is not sufficiently sedated. Not all speech indicates lack of sufficiency of sedation such as murmuring or snoring sound for instance. However, exclamation speech may be taken to indicate that sedation status is not sufficient for imaging. The speech recognizer may be adjusted to identify exclamations in particular. Embodiments where the speech recognizer is adjusted to recognize other types of utterance for reliable sedation status determination are also envisaged.

It will be understood again that each of the above described embodiments of the RMC may be used singly or in any combination or in sub-combination, as required. If more than one of the above embodiments are used in combination, the respective measurement signals may be processed in a multi-channel configuration in which the plurality of response signals are processed together, for example in a machine learning component. The combined signals maybe consolidated into a common score (e.g., average, weighted average or other) to assess the sedation status.

Reference is now made to the flow chart in Fig. 4 which shows steps of a method of supporting an imaging operation. The method steps may underlie operation of the system SYS as discussed above in Figs. 1-3, but it will be understood that the method is not necessarily tied to the architectures outlined in Figs. 1-3, and the following teaching may also be understood as one in its own right.

At step S405 a sedative is administered to a patient, before an intended imaging session.

After a suitable delay, which will depend on properties of the sedative, at step S410 a chemoreceptor stimulus is caused in the patient who resides in or at an imaging apparatus. The chemoreceptor stimulus may be caused by applying electric current or voltage to the patient's chemoreceptor system or may be achieved by supplying chemicals such as odorants or tastants to the patient's nose or mouth, respectively.

At step S420 a response is measured of the patient to the stimulus caused in step S410.

At step S430, based on the measured response, a sedation status/level of the patient it is established for the purposes of imaging the patient. In other words, the established sedation status may be used to inform a user of the imaging apparatus by visual, acoustic or other sensory means that a sufficient level has or has not been achieved. In the latter case an alert signal may be issued, whilst in the former case an affirmatory signal may be used or no signal is issued at all in that case.

In an automated embodiment, the method further comprises a step S440 to actively modify the imaging procedure.

Actively modifying may include halting an ongoing image acquisition procedure or initiating an image acquisition. In addition, a planned imaging acquisition may be delayed. In addition, or instead, the imaging procedure is re-started once it is decided that the sufficient sedation level has been achieved.

In other words, the steps S405-S430 may be repeated to monitor the sedation level over a period of time in a control loop. In particular, the (or a different) sedative may be reapplied based on the established sedation status, to so exercise sedation status control.

The stimulus in step S410 may be administered in a single spurt or may be administered repeatedly over time in quanta of different intensity and/or concentration. This avoids patient adaptation. The tastant or odorant may be applied in gaseous or aerosol form or in liquid form as required. The stimulant may be administered through a nozzle or through a tube or face mask, or through a portable device that is placed into the mouth or nose of the patent.

The stimulus may be caused by administering a chemical that is the tastant or odorant, but may also be achieved in embodiments artificially by applying an electric current to the taste buds of the patient.

For tastant delivery, it may be advantageous to avoid saturating the taste buds. For odorant delivery, avoiding saturating the olfactory system is recommend. In addition, removal of residue of previously administered odorants from the nose area may be advantageous. Such removal may be done by using a vacuum suction device mounted in the vicinity around the patient's nose and operable in alternation with the stimulant deliver component SDC.

The response measuring at step S420 may be achieved by based on image analysis, on measuring vital signs, and/or on measuring a GSR signal or other.

If the monitoring of the response is image based the step S420 may include analyzing the image for motion artifacts. In some embodiments the image is analyzed for facial expressions that are known to represent a sedation level.

In embodiments, step S430 is based on a machine learning algorithm. In general, the step S430 as implemented by the decision logic includes a signal analyzer that analyzes the respective response measurement signal for patterns to establish the sedation status or level. The signals analyzer is adapted to the nature of the response measurement which may be any of the above mentioned data types, such as an image (eg, of the face or other body part), vital sign, GSR, etc. The sedation status/level may be binary, that is, the it represents whether the patient is or is not sufficiently sedated. Alternatively, the sedation level/status is more refined and includes more graduations that range from sedated to partially sedation to not sedated. The analysis outcome for sedation status or levels may be provided in terms of a probability density or probability distribution over the sedation levels or status.

In embodiments, the image analyzer, or signal analyzer more generally, of the decision logic DL may include a pre-trained machine learning component MLC. Neural-networks or support vector machines or other machine learning algorithms may be used to achieve this.

We now turn to two more operational aspects of the decision logic DL or step S420 more generally. One operational aspect (referred to herein as the "response problem") concerns the correct association between observed response and a decision of sufficient or insufficient sedation level. The other operational aspect (referred to herein as the "stimulus problem") concerns the correct association between the patient's characteristics and the stimulus to be delivered that would allow one to reliable distinguish between sedation level (sufficient or not).

Both operational aspects could be addressed in principle by running experiments on a set of individuals of sufficient size drawn from the sample space that is the population of interest. This is essentially a statistical task and a set of suitable thresholds could be derived using "classical" statistical methods to address the two functional aspects. In addition, or instead, the matter may be looked at as problem of using machine learning ("ML") algorithms. Conceptually, the two above mentioned associations maybe understood as latent mapping between relevant spaces, and the ML algorithm can be used to learn approximate representations (eg, parameterized models such as NN or other) for these mappings from training data: one mapping spaces to learn respective latent mappings $F$, $G$ that underlie the two operational aspects:

$$\mathcal{R} \xrightarrow{F} \mathcal{D} \qquad (1)$$

$$\mathcal{P} \xrightarrow{G} S \qquad (2)$$

wherein:

$\mathcal{R}$ is the space of responses;

$\mathcal{D}$ is the decision space. This maybe binary $\mathcal{D} = \{1,0\}$, where symbol '1' codes for sufficient sedation and symbol '0' codes for insufficient sedation level; Alternatively, decision space may include more than two elements to code for more than two sedation levels. $\mathcal{D}$ may be discrete or continuous;

$\mathcal{P}$ is the space of patent characteristics; and
$S$ is the stimulant space.

The above introduced conceptual spaces may be suitably coded as vector spaces, such as

$\mathcal{P} \ni \vec{p} = (p_1, p_2, ..., p_j, ... p_N)$, with each entry representing a measurable characteristic, such as age, sex, weight, height, etc. The stimulus space $S$ may include a coding of taste or smell in terms of material, concentrations, volume and/o in terms of basic components, such as the five base tastes, explored in more detail below.

The response space $\mathcal{R}$, may be conceptualized as an the space of images (of a given size (m x n), the space of vital sign or GSA measurements, etc, depending on which of the above discussed embodiments of the response measurement component RMC is chosen.

The mappings F,G are latent in that are in general unknown but a parameterized from may still be learned by applying ML algorithms to a set of training data. In supervised schemes, the training data may be labelled by a human expert reviewing historic data obtained by experimentation or by retrieving records from medical or medical databases system such as PACS or non-medical databases.

Particular reference is now made to Fig. 5 that shows a flow chart of a method of training a machine learning component as may be used in the decision logic DL mentioned above to address the response problem. Any machine learning algorithm can be used, supervised or unsupervised. In particular, the following machine learning algorithms are envisaged: neural-network based, such as convolutional neural networks-based, in particular with one or more hidden layers ("deep learning"). Other algorithms or setups include support vector machines, decision trees and/or regression or classification methods. The machine learning task to be trained for is essentially one of classification, in particular to learn the latent mapping $F$.

More particularly, at step S510 training data is provided.

The training data can be obtained from historical data sets or can be obtained in experiments conducted with a representative sample of patients.

In supervised learning the training data needs to be labeled and comprises pairs of training input data each associated with a label. In the present case, the training input data includes stimulus response measurements, each labeled by a human expert. The label may be binary and hence codes for whether or not the respective measurement constitutes a sufficient or insufficient sedation level, or the level may be classified in more than two levels, discretely or continuously. In one exemplary embodiment, the human expert reviews images of facial expressions drawn from a representative sample of face, including faces of male and female individuals of different age groups, etc. The human expert then decides whether the given image is one that represents a facial expression of a sedated or non-sedated person. Instead of a binary scheme, more classification levels may be used instead.

Stimulus measurements in terms of imagery of facial expression or other body parts are merely according to one embodiment. Functional brain imagery that captures brain activity may be analyzed by the human export instead or in addition to the imagery, such GSR, vital sign, speech or others as discussed above.

In unsupervised learning schemes no specific labeling may be required.

At step S520, the machine learning algorithm is then applied to the training data, preferably based on a parameterized model. In particular, parameters of a machine learning model are adapted in an optimization procedure based on the training data. The outcome of step S520 is in some embodiments a parameterized model or functional expression that can accept an input to produce an output. A neural network model with adjusted weights is one embodiment of an outcome of step S520. Non-parametric methods may also be used instead in embodiments for step S520.

At step S530 the model, after training, may then be provided for deployment to correctly predict, based on a newly received stimuli, whether or not this represents an instance of sufficient or insufficient sedation level.

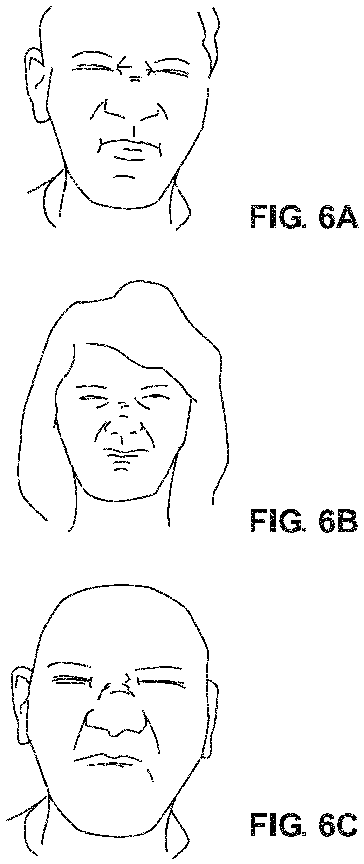

Figs.6A-6C shows representative training image data of facial expressions as may be measured, for instance by a camera with facial recognition, after administration of odorant or tastant. The examples constitute instances where no sufficient sedation level has been achieved. Preferably the training data further includes instances that does show a sufficient sedation level, with more relaxed facial features.

More generally, facial expression are movements of facial skin caused by the brain instruction contractions dilation of subdermal muscles, which include in particular eye squinting, nose pinching or wrinkling or throwing folds, changing the shape of one mouth by wide open, lip eversion, curling, lip motion, pursed and puckering, etc.

A similar approach as explained above may be used instead to learn the mapping $G$ which will now be discussed in more detail, with reference to Fig. 7. This shows a flow chart for a method of chemoreceptor stimulus based control to address the above mentioned "stimulus problem". In particular, this method addresses the problem of providing to a given patient the correct stimulus level that can be expected to allow reliable determination of the actual sedation status. This is essentially a calibration task as different patients may react differently to different stimulant levels.

For instance, elderly patients may require generally a higher level of stimulants to establish reliably whether or not there is a sufficient sedation level as compared to younger patients.

Conceptually, there is an unknown and perhaps complex mapping between patient characteristics and the right level of stimulant level to be provided. This mapping can be learned by machine learning as previously discussed in Fig. 4.

Referring now to the case of stimulus by tastant, it is known that each taste sensation can be decomposed from five base tastants including sweet ($x_1$), umami [savory] ($x_2$), sour ($x_3$) or salty and ($x_4$) bitter ($x_5$), with $x_i$ indicating amounts or concentration. This can be formalized as taste stimulus $T$ for patient $i \rightarrow T = (x1,x2,x3,x4,x5)$. This taste combination is the one that would yield the highest response for a given patient: $G(\vec{p}) = (x_1, x_2, x_3, x_4, x_5)$.

A taste sensation, or response, may thus be visualized conceptually as a point ($x_1,x_2,x_3,x_4,x_5$) in a five dimensional space, the stimulant space $S$, which may now be referred to more specifically as the taste space. Equally, patient characteristics such as sex, weight, age etc may be conceptualized as points in the one or higher dimensional patient space $\mathcal{P}$. The Cartesian products of those two spaces, the taste space and the patient space, may first be conceptualized as a 5+$p$ dimensional space, with $p$ being the number of patient characteristics to be considered, and hence the dimension of patient space $\mathcal{P}$.

It may then be expected that the right level of stimuli may be represented in this combined space $\mathcal{P}$ x $S$ as clusters corresponding to different patient characteristics. This space can be analyzed by machine learning algorithms, in clustering algorithms, to find clusters. This clustering may be done in an unsupervised setup. A support vector machine (SWM) approach may be used. The clusters so found represent the right stimulus, in this case taste sensation, for the patient given their characteristics.

In more detail, at step S710 training data is received that comprises items of patient data, each associated with a particular tastant data $T$. The tastant data $T$ is the specific base tastant ($xi$) combination that yields the highest or sufficiently high, response for a patient with bio-characteristics $\vec{p}$.

At step S720 a machine learning algorithm is applied to the training data to learn the latent relationship $G$ between the patient data and the stimulant data T. This relationship may be learned using, as mentioned, clustering algorithms or may be learned using a neural-network model and treating the learning task as a one of regression rather than clustering.

Steps S710 and S720 maybe understood to form the calibration phase.

Once the relationship G has been learned, method flow may then proceed to step S730, where, based on the learned relationship, a new stimulant data can be predicted based on new patient data for a given patient to be imaged.

The stimulation delivery component may hence be adjusted based on the predicted stimulant data. More particularly, the stimulant data may prescribe a specific combination of the base tastes which can be expected to yield the most reliable results to establish the true sedation level of the patient.

At step S740 the patient is then so stimulated based on the new stimulant data to establish the sedation status.

In case where supervised learning is used, experiments may need to be conducted with patients drawn from a representative sample from the patient population. Each patient is then given samples from taste space represented by a different combination of tastants. In this manner, an approximation of the highest response level, or sufficiently high, response level may be found. A human expert, or observer, evaluates the associated response data, such as imagery of facial expression or any of the other discussed measurements signals and labels the accordingly.

The training data so obtained in this experimentation phase may thus comprise pairs of patient data on the one hand and the most effective tastant combination on the other. In this case, the tastant combinations may form the respective targets associated with the respective patient data.

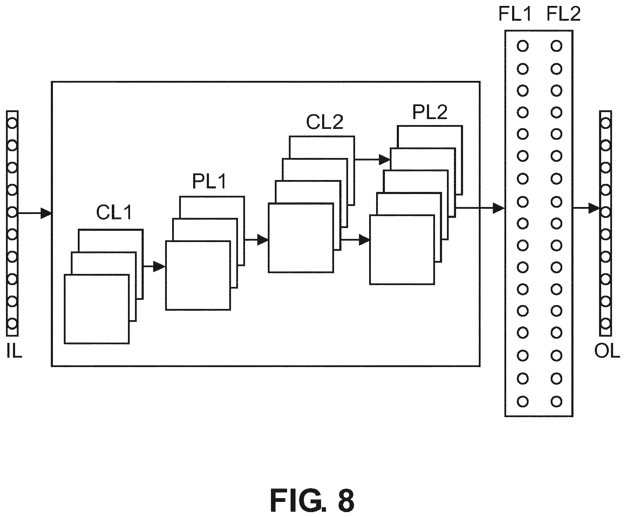

A neural-network model as shown in Fig. 8 below may then be used to learn the relationship G. Once the neural-network is fully trained it can be used to predict the best tastant combination by applying newly given patient data to the network at its input layer to receive at the output layer through forward propagation a proposed combination of base tastants. In other words, the output layer in this embodiment may be formed by a five dimensional vector representing the respective point in taste space. The dimension of the input layer with depend on the dimension of patient vector $\vec{p}$.

As mentioned earlier, the stimulant may be delivered based on a delivery curve in a defined sequence. The body reaction is measured with respect to reaction strength. The stimulants can be varied in a sawtooth taste concentration level profile in time with no or neutral taste between in between response measurements. This may be understood by way of example, in administering "yoghurt" as a neutralizer between administration of spicy dishes, or the delivery of a more benign fragrance in between delivery of two pungent smell samples. A defined sequence of fragrance or taste intensity with different gradients is applied for sensitivity control. This approach with a varying delivery curve may be used in the above mentioned experimentation stage to find the best stimulant for a given patient in terms of response strength used in an automatic procedure. In addition or instead, the varying delivery curve may be used to measure sedation level depth.

In Fig. 8 a basic architecture for a neural-network model is represented that may be used in embodiments to learn the relationship between stimulant data and patient characteristics. Specifically, Fig. 8 is a schematic diagram of a convolutional neural-network ("CNN"). However, this is not at the exclusion of alternative embodiments such as support vector machines, linear regression algorithms, decision trees, or otherwise, all equally envisaged herein.

The CNN is operable in two modes: "training mode/phase" and "deployment mode/phase". In training mode, an initial model of the CNN is trained based on a set of training data to produce a trained CNN model. In deployment mode, the pre-trained CNN model is fed with non-training, new data, to operate during normal use. The training mode may be a one-off operation or this is continued in repeated training phases to enhance performance. All that has been said so far in relation to the two modes is applicable to any kind of machine learning algorithms and is not restricted to CNNs or, for that matter, NNs.

The CNN comprises a set of interconnected nodes organized in layers. The CNN includes an output layer OL and an input layer IL. The CNN has preferably a deep learning architecture, that is, in between the OL and IL there is at least one, preferably two or more, hidden layers. Hidden layers may include one or more convolutional layers CL1, CL2 ("CL") and/or one or more pooling layers PL1, PL2 ("PL") and/or one or more fully connected layer FL1, FL2 ("FL"). CLs are not fully connected and/or connections from CL to a next layer may vary but are in generally fixed in FLs.

Nodes are associated with numbers, called "weights", that represent how the node responds to input from earlier nodes in a preceding layer.

The set of all weights defines a configuration of the CNN. In the learning phase, an initial configuration is adjusted based on the training data using a learning algorithm such as forward-backward ("*FB*")-propagation or other optimization schemes, or other gradient descent methods. Gradients are taken with respect of the parameters of the objective function.

The training mode is preferably supervised, that is, is based on annotated training data. Annotated training data includes pairs or training data items. For each pair, one item is the training input data and the other item is target training data known a priori to be correctly associated with its training input data item. This association defines the annotation and is preferably provided by a human expert.

In training mode, preferably multiple such pairs are applied to the input layer to propagate through the CNN until an output emerges at OL. Initially, the output is in general different from the target. During the optimization, the initial configuration is readjusted so as to achieve a good match between input training data and their respective target for all pairs. The match is measured by way of a similarity measure which can be formulated in terms of on objective function, or cost function. The aim is to adjust the parameters to incur low cost, that is, a good match.

More specifically, in the NN model, the input training data items are applied to the input layer (IL) and passed through a cascaded group(s) of convolutional layers CL1, CL2 and possibly one or more pooling layers PL1, PL2, and are finally passed to one or more fully connected layers. The convolutional module is responsible for feature based learning (e.g. identifying features in the patient characteristics and context data, etc.), while the fully connected layers are responsible for more abstract learning.

The exact grouping and order of the layers as per Fig 8 is but one exemplary embodiment, and other groupings and order of layers are also envisaged in different embodiments. Also, the number of layers of each type (that is, any one of CL, FL, PL) may differ from the arrangement shown in Fig 8. The depth of the CNN may also differ from the one shown in Fig 8. All that has been said above is of equal application to other NNs envisaged herein, such as fully connected classical perceptron type NN, deep or not, and recurrent NNs, or others. In variance to the above, unsupervised learning or reinforced leaning schemes may also be envisaged in different embodiments.

The labeled training data, as envisaged herein may need to be reformatted into structured form. As mentioned, the annotated training data may be arranged as vectors or matrices or tensor (arrays of dimension higher than 2). This reformatting may be done by a data pre-processor module (not shown), such as scripting program or filter that runs through patient records of the HIS of the current facility to pull up a set of patient characteristics.

The training data sets are applied to an initially configured CNN and is then processed according to a learning algorithm such as the FB-propagation algorithm as mentioned before. At the end of the training phase, the so pre-trained CNN may then be used in deployment phase to compute the decision support information for new data, that is, newly acquired copy images not present in the training data.

Some or all of the above mentioned steps may be implemented in hardware, in software or in a combination thereof. Implementation in hardware may include a suitably programmed FPGA (field-programmable-gate-array) or a hardwired IC chip. For good responsiveness and high throughput, multi-core processors such as GPU or TPU or similar may be used to implement the above described training and deployment of the machine learning model, in particular for NNs.

One or more features disclosed herein may be configured or implemented as/with circuitry encoded within a computer-readable medium, and/or combinations thereof. Circuitry may include discrete and/or integrated circuitry, application specific integrated circuitry (ASIC), a system-on-a-chip (SOC), and combinations thereof., a machine, a computer system, a processor and memory, a computer program.

The components of the image processing system SYS may be implemented as software modules or routines in a single software suit and run on a general purpose computing unit PU such as a workstation associated with the imager IA or a server computer associated with a group of imagers. Alternatively, the components of the system SYS may be arranged in a distributed architecture and connected in a suitable communication network.

Some or all components of system SYS may be arranged in hardware such as a suitably programmed FPGA (field-programmable-gate-array) or as hardwired IC chip.

One or more features disclosed herein may be configured or implemented as/with circuitry encoded within a computer-readable medium, and/or combinations thereof. Circuitry may include discrete and/or integrated circuitry, application specific integrated circuitry (ASIC), a system-on-a-chip (SOC), and combinations thereof., a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. A system (SYS) for imaging support, comprising:

   - a stimulus delivery component (SDC) configured to cause a chemoreceptor stimulus in a patient residing in or at an imaging apparatus (IA);
   - a response measuring component (RMC) configured to measure a response of the patient to the stimulus; and
   - a decision logic (DL) configured to establish, based on the measured response, a sedation status of the patient for the purpose of imaging the patient.

2. System of claim 1, wherein the decision logic (DL) is further to provide, based on the established sedation status, at least one of i) a control signal to control the imaging apparatus and/or ii) an indication on the sedation status and/or on how to operate the imaging apparatus.

3. System of claim 1 or 2, wherein the causing by the stimulus delivery component (SDC) of the stimulus comprises any one or more of: i) providing a substance to the patient capable of stimulating a chemoreceptor of the patient or ii) applying an electrical signal to a chemoreceptor of the patient.

4. System of any one of the previous claims, wherein the decision logic comprises a pre-trained machine learning component (MLC).

5. System of any one of the previous claim, wherein the stimulus delivery component (SDC) comprises any one or more of: i) a portable device configured for oral placement and configured to electrically induce a taste sensation in a patient by having electrodes of the device in contact with at least a part of the patient's tongue when so placed, and configured to apply a voltage across the electrodes driven by a power source ii) a portable device configured for oral placement, having at least one container including a tastant or odorant, the device configured to release a quantum of said tastant upon the device receiving a trigger signal, iii) a tube arrangement in fluid communication with a container including a tastant or odorant, and a pump configured to pump a quantum of the tastant or odorant from the container, through the tube arrangement, to a terminal end thereof, so as to dispense the quantum of tastant or odorant at the patients mouth or nose, iv) a jet-delivery system including a nozzle through which an odorant is delivered as an aerosol.

6. System of claim 5, wherein the power source is based on inductive coupling.

7. System of any one of the previous claims, wherein the response measuring component (RMC) comprises a sensor arrangement capable of measuring any one or more in relation to the patient: i) a galvanic skin response [GSR] signal, ii) a facial gesture, iii) a body gesture, iv) an uttered sound, v) a vital sign.

8. System of any one of the previous claims, wherein the stimulus is caused in a sequence of different intensities.

9. Method (500) of training the machine learning component (MLC) as per claim 4.

10. Method (400) of imaging support, comprising the steps of:

   - causing (S410) a chemoreceptor stimulus in a patient residing in or at an imaging apparatus (IA);
- measuring (S420) a response of the patient to the stimulus; and
- establishing (S430), based on the measured response, a sedation status of the patient for the purpose of imaging the patient.

11. A method (700) for chemoreceptor stimulus based sedation control, comprising the steps of:

- receiving (S710) training data comprising patient data and stimulant data, the stimulant data comprising a combination of different base tastes; and
- applying (S720) a machine learning algorithm to the training data to learn a relationship between the patient data and the stimulant data.

12. Method of claim 11, further comprising:

- predicting (S730), based on the learned relationship, new stimulant data given new patient data of a patient to be imaged; and
- stimulating (S740) the patient based on the new stimulant data to establish a sedation status of the patient.

13. An imaging arrangement (IA), comprising:

- a system of any one of previous claims 1-8;
- the imaging apparatus (IA).

14. A computer program element, which, when being executed by at least one processing unit (PU), is adapted to cause the processing unit (PU) to perform the method as per any one of claims 9-12.

15. A computer readable medium having stored thereon the program element of claim 14.

**FIG. 1**

**FIG. 2**

FIG. 3A

FIG. 3B

**FIG. 4**

**FIG. 5**

FIG. 6A

FIG. 6B

FIG. 6C

S710

S720

S730

S740

**FIG. 7**

**FIG. 8**

Europäisches
Patentamt

European
Patent Office

Office européen
des brevets

**EUROPEAN SEARCH REPORT**

Application Number

EP 19 18 4582

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 681 121 A (KOBAL GERD [DE]) 21 July 1987 (1987-07-21) <br><br> * FIELD OF THE INVENTION; column 1, line 5 - line 20 * <br> * column 2, line 16 - line 24 * <br> * column 3, line 39 - column 4, line 6; figure 1 * <br> * column 4, line 12 - line 26 * <br> ----- | 1-3,5,7, 8,10, 13-15 | INV. <br> A61B5/00 <br> A61M16/01 <br><br> ADD. <br> A61B5/11 |
| A | G. VON BÉKÉSY: "Sweetness produced electrically on the tongue and its relation to taste theories", JOURNAL OF APPLIED PHYSIOLOGY, vol. 19, no. 6, 1 November 1964 (1964-11-01), pages 1105-1113, XP055646958, US <br> ISSN: 8750-7587, DOI: 10.1152/jappl.1964.19.6.1105 <br> * the whole document * <br> ----- | 5 | |
| | | | TECHNICAL FIELDS SEARCHED (IPC) <br><br> A61B <br> A61M |

~~The present search report has been drawn up for all claims~~

| Place of search | Date of completion of the search | Examiner |
|---|---|---|
| The Hague | 8 January 2020 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

Application Number

EP 19 18 4582

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

    see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

    2, 3, 7, 8, 10, 13(completely); 1, 5, 14, 15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 19 18 4582

The Search Division considers that the present European patent application does not comply with the
requirements of unity of invention and relates to several inventions or groups of inventions, namely:

1. claims: 2, 3, 7, 8, 10, 13(completely); 1, 5, 14, 15(partially)

   Method and system to estimate sedation status by
   chemoreceptor stimulus induced response
   ---

2. claims: 4, 9, 11, 12(completely); 1, 14, 15(partially)

   Machine learning implementation of sedation estimation
   ---

3. claims: 6(completely); 5(partially)

   Power source for oral portable device for electrically
   inducing a taste sensation in a patient by electrodes
   ---

## ANNEX TO THE EUROPEAN SEARCH REPORT
## ON EUROPEAN PATENT APPLICATION NO.

EP 19 18 4582

This annex lists the patent family members relating to the patent documents cited in  the above-mentioned European search report.
The members are as contained in the European Patent Office EDP file on
The European Patent Office is in no way liable for these particulars which are merely given for the purpose of information.

08-01-2020

| Patent document cited in search report | | Publication date | Patent family member(s) | | Publication date |
|---|---|---|---|---|---|
| US 4681121 | A | 21-07-1987 | DE | 3501095 A1 | 17-07-1986 |
| | | | FR | 2575916 A1 | 18-07-1986 |
| | | | GB | 2169713 A | 16-07-1986 |
| | | | US | 4681121 A | 21-07-1987 |

EPO FORM P0459

For more details about this annex : see Official Journal of the European Patent Office, No. 12/82